# EUROPÄISCHE PATENTSCHRIFT

(11) **EP 1 704 897 B1**
(45) Veröffentlichungstag und Bekanntmachung des Hinweises auf die Patenterteilung: **19.08.2009**
(21) Anmeldenummer: 06005380.8
(22) Anmeldetag: 16.03.2006
(51) Int. Cl.: A61Q 9/02, A61Q 5/02, A61Q 19/10, A61K 8/04, A61K 8/86, A61K 8/36, A61K 8/34, C11D 1/83

(54) **Schäumbare kosmetische Zusammensetzungen**
Foamable cosmetic compositions
Compositions cosmétiques moussables

(30) Priorität: 22.03.2005 DE 102005013768
(43) Veröffentlichungstag der Anmeldung: 27.09.2006
(73) Patentinhaber: Henkel AG & Co. KGaA, 40589 Düsseldorf (DE)
(72) Erfinder: Lapena, Margarita, 08026 Barcelona (ES); Emmerling, Winfried, 25436 Tornesch (DE); Pujol Galiano, Miracle, 08329 Teià (ES)

(56) Entgegenhaltungen:
- WO-A-02/087519
- US-A- 5 179 122
- US-A- 5 248 495
- US-B1- 6 352 689
- ANONYM: "Polyox Water-Soluble Resins" INTERNET, [Online] Mai 2002 (2002-05), XP002390203 Gefunden im Internet: URL:http://www.in-cosmetics.com/ExhibitorL ibrary/234/POLYOX_Water_Soluble_Resins.pdf > [gefunden am 2006-07-12]

## Beschreibung

Die vorliegende Erfindung betrifft schäumbare kosmetische Zusammensetzungen, insbesondere nachschäumende kosmetische Zusammensetzungen.

Schäume bzw. schaumförmige Zusammensetzungen gehören zu den dispersen Systemen. Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere bei der Reinigung, der Rasur, beispielsweise als Rasierschaum, und der Haarpflege Verwendung.
Derartige Schäume sind nur wenig stabil, weshalb sie üblicherweise innerhalb von etwa 24 Stunden zusammenfallen. Eine Anforderung an kosmetische Zusammensetzungen ist aber, dass diese eine möglichst jahrelange Stabilität besitzen. Diesem Problem wird im allgemeinen dadurch Rechnung getragen, dass der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterlässt einen Schaum.
Sogenannte nachschäumende kosmetische Zusammensetzungen werden zunächst in fließförmiger Form, beispielsweise als Gel, mit Hilfe eines Treibmittels aus einem Aerosolbehäfter auf die Haut aufgetragen. Als Treibmittel dient üblicherweise eine Mischung aus mindestens einem Treibgas mit einem sehr niedrigen Siedepunkt, z. B. Butanen, insbesondere Isobutan, und mindestens einem Treibgas mit einem etwas höheren Siedepunkt, dem "Nachschäummittel", z. B. Pentanen, insbesondere Isopentan und n-Pentan. Beim Verreiben auf der Haut verdunstet das höher siedende Treibmittel durch die Hautwärme und bildet erst nach kurzer Verzögerung den eigentlichen Schaum, beispielsweise einen Rasierschaum.

WO 02/087519 A2 und WO 02/087520 A2 offenbaren nachschäumbare Rasiergele, die Wasser, Seifen sowie weitere Tenside, ein Polysaccharid-Gum und 0,11 - 0,24 Gew.-% eines Polyethylenglycols mit einem Molekulargewicht von mindestens 1 Million Dalton enthalten.
WO 98/33473 offenbart nachschäumbare Rasiergele, die Wasser, Seifen sowie weitere Tenside, Polyvinylpyrrolidon und 0,087 Gew.-% eines Polyethylenglycols mit einem mittleren Molekulargewicht von etwa 4 Million Dalton enthalten.
GB 2151657 A1 offenbart ein nachschäumbares Rasiergel, das Wasser, Seife, gering schäumende ethoxylierte Tenside, ein 2-Acrylamido-2-methylpropansulfonsäure-Polymer und ein Diallyldimethylallylammoniumchlorid-Acrylamid-Copolymer als Polymerverdicker und 0,071 Gew.-% eines Polyethylenglycols mit einem mittleren Molekulargewicht von etwa 4 Millionen Dalton enthält und das frei ist von hydrophoben Coemulgatoren.
Auch andere derartige Zusammensetzungen sind bekannt, die aber alle den Nachteil haben, kosmetisch unelegant zu sein, das heißt, dass beispielsweise der produzierte Schaum ein klebriges Gefühl erzeugt und/oder von ungenügender Konsistenz ist oder aber das Gel so stark verdickt ist, dass es bei der Herstellung ungenügende Abfülleigenschaften aufweist.
US 5248495 offenbart nachschäumbare Rasiergele, deren Tensidsystem zur Verbesserung der Hautverträglichkeit Seife und daneben als einziges nichtionisches Tensid ein Siliconcopolymer-Tensid enthält.
Auch selbstschäumende Duschzusammensetzungen sind im Stand der Technik bekannt. So beschreibt die WO 97/03646 Reinigungszusammensetzungen, die eine Grundformulierung aus mindestens einem Detergens und einem Verdicker enthalten, wobei die Viskosität der Grundformulierung größer als 9.500 cps ist. Da durch Zugabe des Treibmittels zur Grundformulierung die Viskosität des Endprodukts stark herabgesetzt wird, haben diese Formulierungen eine hohe Anfangsviskosität, um eine ausreichend hohe Viskosität des Endproduktes (mit Treibmittel) zu erreichen.

Ein Zusatz an hochmolekularen Polyethylenglycolen verdickt die Zusammensetzung und bildet auf der Haut einen Film, der bei der Rasur das Gleiten der Klinge über die Haut verbessert und somit die Rasur erleichtert. Außerdem verleihen hochmolekulare Polyethylenglycole der Zusammensetzung ein angenehmes Hautgefühl. Ein zu hoher Gehalt an hochmolekularen Polyethylenglycolen führt allerdings zu einer zu hohen Elastizität der Zusammensetzung, was z. B. zu Fädenziehen und einer schlechteren Verteilbarkeit auf der Haut führen kann. Auch kann ein zu hoher Gehalt an hochmolekularen Polyethylenglycolen die Qualität und das Hautgefühl des Schaumes beeinträchtigen. Ein zu hoher Gehalt an hochmolekularen Polyethylenglycolen führt außerdem zu Problemen bei der Herstellung, da sich derartige elastische Zusammensetzungen nur unter größerem Energieeintrag homogenisieren und anschließend nur langsam abfüllen lassen.

Aufgabe der vorliegenden Erfindung war es daher, schäumbare kosmetische Zusammensetzungen, insbesondere nachschäumende Rasierzusammensetzungen, bereitzustellen, die gegenüber dem Stand der Technik verbesserte Anwendungseigenschaften aufweisen. Eine weitere Aufgabe war es, schäumbare kosmetische Zusammensetzungen, insbesondere nachschäumende Rasierzusammensetzungen, bereitzustellen, die gegenüber dem Stand der Technik ein verbessertes Hautgefühl aufweisen. Eine weitere Aufgabe war es, schäumbare kosmetische Zusammensetzungen, insbesondere nachschäumende Rasierzusammensetzungen, mit gegenüber dem Stand der Technik verbesserten Schaumeigenschaften bereitzustellen. Eine weitere Aufgabe war es, schäumbare kosmetische Zusammensetzungen, insbesondere nachschäumende Rasierzusammensetzungen, mit gegenüber dem Stand der Technik verbesserten Produktions- und Abfülleigenschaften bereitzustellen.

Überraschend wurde gefunden, dass eine Verringerung des Gehaltes an hochmolekularen Polyethylenglycolensowie ein Zusatz an Kieselsäure zusammen mit einer spezifischen Kombination aus Seifen, nichtionischen Tensiden, hydrophoben Coemulgatoren, Polyolen und Polymerverdickern die Nachteile des Standes der Technik beseitigt.

Gegenstand der vorliegenden Erfindung sind kosmetische Zusammensetzungen, die mit einem nachschäumenden Treibmittel aufgeschäumt werden können und die Wasser, mindestens eine C₁₀ - C₂₄-Alkancarbonsäure, mindestens ein Neutralisierungsmittel für die Alkancarbonsäure/n, mindestens ein nichtionisches Tensid, mindestens einen hydrophoben Coemulgator, mindestens ein wasserlösliches C₃ - C₉-Polyol, mindestens ein verdickendes Polymer, das kein Polyethylenglycol darstellt, wobei nicht gleichzeitig mindestens ein 2-Acrylamido-2-methylpropansulfonsäure-Polymer und mindestens ein Diallydimethylallylammoniumchlorid-Acrylamid-Copolymer als verdickende Polymere enthalten sind, mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung, und weiterhin Kieselsäure enthalten.
Das erfindungsgemäß charakterisierende Polyethylenglycol mit einem mittleren Molekulargewicht von mindestens 300.000 Dalton hat die allgemeine Strukturformel H-(OCH₂CH₂-)ₙOH.

Es ist erfindungsgemäß vorteilhaft, wenn die Zugabe des Sekundärtreibmittels, also des höher siedenden nachschäumenden Treibmittels, bei oder vor der Abfüllung der Zusammensetzung in den Aerosolbehälter erfolgt. Wählt man die Abfüllung des Endproduktes so, dass Anfangsformulierung und Sekundärtreibmittel gemeinsam abgefüllt werden und dass dabei eine intensive Durchmischung erfolgt, so lassen sich sehr hohe Abfüllgeschwindigkeiten erreichen, da die Mischung eine niedrige Viskosität hat.
Die erfindungsgemäßen Zusammensetzungen stellen in jeglicher Hinsicht überaus befriedigende Präparate dar. Es war insbesondere überraschend, dass die aus den erfindungsgemäßen selbstschäumenden bzw. schäumbaren Zusammensetzungen hergestellten Schäume - auch bei einem ungewöhnlich hohen Gasvolumen - außerordentlich stabil sind. Dementsprechend eignen sich Zusammensetzungen im Sinne der vorliegenden Erfindung ganz besonders, um als Grundlage für Produktformen mit vielfältigen Anwendungszwecken zu dienen.
Die erfindungsgemäßen Zusammensetzungen und daraus erhältliche Schäume weisen sehr gute sensorische Eigenschaften auf, wie beispielsweise Verteilbarkeit auf der Haut, sensorischer Eindruck, Volumen und Konsistenz des erzeugten Schaums und - besonders wichtig für die Verwendung als Rasierhilfsmittel - gutes Gleitvermögen der Rasierklinge über die Haut ("lubricity").
Aus den erfindungsgemäßen Zusammensetzungen sind feinblasige, reichhaltige, cremige Schäume von hervorragender kosmetischer Eleganz erhältlich. Weiterhin sind aus den erfindungsgemäßen Zusammensetzungen besonders gut hautverträgliche Zusammensetzungen erhältlich, die sich besonders gut auf der Haut verteilen lassen.

Erfindungsgemäß bevorzugte C₁₀ - C₂₄-Alkancarbonsäuren sind ausgewählt aus linearen und verzweigten, gesättigten und ungesättigten Alkancarbonsäuren, deren Alkylkette mit funktionellen Gruppen, beispielsweise OH-Gruppen, substituiert sein kann. Bevorzugt sind Decansäure, Decensäure, Undecansäure, Undecensäure, Dodecansäure, Dodecensäure, Tridecansäure, Tridecensäure, Myristinsäure, Tetradecensäure, Pentadecansäure, Pentadecensäure, Palmitinsäure, Hexadecensäure, Stearinsäure, Ölsäure, Linolsäure, Linolensäure, Isostearinsäure, Ricinolsäure, hydrierte Ricinolsäure (= 12-Hydroxystearinsäure), Eicosansäure, Eicosensäure, Arachidonsäure und Behensäure. Erfindungsgemäß besonders bevorzugte C₁₀ - C₂₄-Alkancarbonsäuren sind ausgewählt aus Decansäure, Undecansäure, Dodecansäure, Tridecansäure, Myristinsäure, Palmitinsäure, Stearinsäure, Isostearinsäure, Eicosansäure und Behensäure. Außerordentlich bevorzugt sind Palmitinsäure und Stearinsäure sowie Mischungen hiervon.
Bevorzugte erfindungsgemäße Zusammensetzungen sind dadurch gekennzeichnet, dass sie mindestens eine C₁₀- C₂₄-Alkancarbonsäure in einer Gesamtmenge von 5-25 Gew.-%, bevorzugt 8-20 Gew.-% und besonders bevorzugt 10-16 Gew.-%, enthalten, jeweils bezogen auf das Gesamtgewicht der treibmittelfreien Zusammensetzung.

Die mindestens eine C₁₀ - C₂₄-Alkancarbonsäure liegt in den erfindungsgemäßen Zusammensetzungen ganz oder teilweise in mit üblichen Alkalien (wie insbesondere bevorzugt Natrium- und/oder Kaliumhydroxid, Natrium- und/oder Kaliumcarbonat sowie Mono- und/oder Triethanolamin) neutralisierter Form vor und wird in dieser Form auch als Seife bezeichnet. Bevorzugte Seifen sind Natriumstearat, Kaliumstearat, Triethanolaminstearat, Natriumisostearat, Kaliumisostearat, Triethanolaminisostearat, Natriumpalmitat, Kaliumpalmitat, Triethanolaminpalmitat, Natriumbehenat, Kaliumbehenat, Triethanolaminbehenat, Natriummyristat, Kaliummyristat und Triethanolaminmyristat sowie Mischungen hiervon. Besonders bevorzugt sind Triethanolaminstearat, Triethanolaminpalmitat sowie Mischungen hiervon, insbesondere solche Mischungen mit einem Gewichtsverhältnis von Palmitat zu Stearat von 1,5:1 bis 3:1, bevorzugt 2:1 bis 7:3.

Der Neutralisierungsgrad der C₁₀ - C₂₄-Alkancarbonsäure/n beträgt bevorzugt mindestens 80 %, besonders bevorzugt mindestens 90 %, außerordentlich bevorzugt mindestens 95 % und weiterhin außerordentlich bevorzugt 100 %.

Erfindungsgemäß bevorzugte nichtionische Tenside sind
- Aminoxide,
- Fettsäurealkanolamide,
- Anlagerungsprodukte von Ethylenoxid und/oder Polyglycerin an Fettsäureamide, Fettsäurealkanolamide, Fettamine und Fettsäure-N-alkylglucamide,
- Anlagerungsprodukte von 2 bis 50 Mol Ethylenoxid und/oder 0 bis 5 Mol Propylenoxid an lineare und verzweigte, gesättigte und ungesättigte Fettalkohole mit 8 bis 30 C-Atomen, an Fettsäuren mit 8 bis 30 C-Atomen und an Alkylphenole mit 8 bis 15 C-Atomen in der Alkylgruppe,
- Alkylpolyglycoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglycoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglycoside, bei denen R
- im wesentlichen aus C₈- und C₁₀-Alkylgruppen oder
- im wesentlichen aus C₁₂- und C₁₄-Alkylgruppen oder
- im wesentlichen aus C₈- bis C₁₆-Alkylgruppen oder
- im wesentlichen aus C₁₂- bis C₁₆-Alkylgruppen oder
- im wesentlichen aus C₁₆- bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, ldose, Talose und Sucrose. Bevorzugte Zuckerbausteine sind Glucose, Fructose, Galactose, Arabinose und Sucrose; Glucose ist besonders bevorzugt.
Die erfindungsgemäß verwendbaren Alkylpolyglycoside enthalten durchschnittlich 1,1 bis 5 Zuckereinheiten. Alkylpolyglycoside mit x-Werten (= Oligomerisierungsgrad) von 1,1 bis 2,0 sind bevorzugt. Besonders bevorzugt sind Alkylglycoside, bei denen x 1,1 bis 1,8 beträgt.
Auch die alkoxylierten Homologen der genannten Alkylpolyglycoside können erfindungsgemäß eingesetzt werden. Diese Homologen können durchschnittlich bis zu 10 Ethylenoxid- und/oder Propylenoxideinheiten pro Alkylglycosideinheit enthalten;
- Fettsäure-N-alkylglucamide,
- mit einem Methyl- oder C₂₋₆-Alkylrest endgruppenverschlossene Anlagerungsprodukte von 2 bis 50 Mol Ethylenoxid und/oder 0 bis 5 Mol Propylenoxid an lineare und verzweigte C₈₋₃₀-Fettalkohole, an C₈₋₃₀-Fettsäuren und an C₈₋₁₅-Alkylphenole, z.B. die von der Firma Cognis erhältlichen Handelsprodukte Dehydol^{®} LS und Dehydol^{®} LT,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Ethylenoxid-Anlagerungsprodukte an Fettsäureester von Polyolen wie Glycerin und Trimethylolpropan, z. B. die Handelsprodukte Hydagen^{®} HSP oder Sovermol^{®} (Cognis),
- alkoxylierte Triglyceride,
- alkoxylierte Fettsäurealkylester der Formel R¹CO-(OCH₂CHR²)ₙOR³, in der R'CO für einen linearen oder verzweigten, gesättigten und/oder ungesättigten C₆₋₂₂-Acylrest, R² für Wasserstoff oder eine Methylgruppe, R³ für lineare oder verzweigte C₁₋₄-Alkylreste und n für Zahlen von 1 bis 20 steht,
- Hydroxymischether, wie sie beispielsweise in der DE-OS 19738866 beschrieben sind,
- Anlagerungsprodukte von Ethylenoxid an Sorbitanfettsäureester wie beispielsweise die Polysorbate,
- Zuckerfettsäureester und Anlagerungsprodukte von Ethylenoxid und Polyglycerin an Zuckerfettsäureester,
- Gemische aus Alkyl-(oligo)-glucosiden und Fettalkoholen, zum Beispiel als Handelsprodukte Montanov^{®} 68 oder Montanov^{®} 202 erhältlich,
- Sterine, also Steroide, die am C-Atom 3 des Steroid-Gerüstes eine Hydroxylgruppe tragen und sowohl aus tierischem Gewebe (Zoosterine, z. B. Cholesterin, Lanosterin) wie auch aus pflanzlichen Fetten (Phytosterine, z. B. Ergosterin, Stigmasterin, Sitosterin) und aus Pilzen und Hefen (Mykosterine) isoliert werden;
- Phospholipide, die z.B. als Lecithine bzw. Phosphatidylcholine aus z.B. Eidotter oder Pflanzensamen (z.B. Sojabohnen) gewonnen werden;
- Polyglycerine und Polyglycerinderivate, beispielsweise Polyglycerinpoly-12-hydroxystearat (Handelsprodukt Dehymuls^{®} PGPH der Firma Cognis).

Besonders bevorzugte nichtionische Tenside sind Fettsäurealkanolamide, insbesondere die Monoethanol- und Diethanolamide von linearen C₆ - C₂₂-Alkancarbonsäuren und deren Mischungen, bevorzugt von linearen C₈-C₁₈-Alkancarbonsäuren und deren Mischungen, insbesondere Lauramide DEA und Cocamide DEA. Weitere besonders bevorzugte nichtionische Tenside sind Anlagerungsprodukte von 1-10 Mol Ethylenoxid an Fettsäureamide, insbesondere an die Amide von linearen C₆-C₂₂-Alkancarbonsäuren und deren Mischungen, insbesondere PEG-4 Rapeseedamide. Weitere besonders bevorzugte nichtionische Tenside sind Anlagerungsprodukte von 2 bis 50 Mol, bevorzugt 5 - 40 Mol Ethylenoxid an lineare und verzweigte, gesättigte und ungesättigte C₈-C₃₀-Alkanole und C₈ - C₃₀-Alkenole, bevorzugt C₁₂ - C₁₈-Alkanole und C₁₂ - C₁₈-Alkenole, insbesondere Laureth-23 und Oleth-20.
In einer besonders bevorzugten Ausführungsform der Erfindung ist als nichtionisches Tensid mindestens ein Fettsäurediethanolamid enthalten.
In einer weiteren besonders bevorzugten Ausführungsform der Erfindung ist als nichtionisches Tensid eine Mischung aus mindestens einem Fettsäurediethanolamid und mindestens einem Anlagerungsprodukt von 5-40 Mol Ethylenoxid an lineare und verzweigte, gesättigte und ungesättigte C₁₂ - C₁₈-Alkanole und C₁₂ - C₁₈-Alkenole enthalten.
In einer weiteren besonders bevorzugten Ausführungsform der Erfindung ist als nichtionisches Tensid eine Mischung aus mindestens einem Fettsäurediethanolamid und Laureth-23 enthalten. In einer weiteren besonders bevorzugten Ausführungsform der Erfindung ist als nichtionisches Tensid eine Mischung aus mindestens einem Fettsäurediethanotamid und Oleth-20 enthalten.

Das/die nichtionische/n Tensid/e ist/sind in den erfindungsgemäßen Zusammensetzungen bevorzugt in Gesamtmengen von 0,1-5 Gew.-%, besonders bevorzugt 0,3 - 3 Gew.-% und außerordentlich bevorzugt 0,5 - 2 Gew.-% oder 1,0-1.5 Gew.-%, enthalten, jeweils bezogen auf das Gesamtgewicht der treibmittelfreien Zusammensetzung.

Der Wassergehalt der erfindungsgemäßen Zusammensetzungen beträgt bevorzugt 60 - 95 Gew.-%, besonders bevorzugt 70 - 90 Gew.-% und außerordentlich bevorzugt 75 - 85 Gew.-%, jeweils bezogen auf das Gesamtgewicht der treibmittelfreien Zusammensetzung.

Die erfindungsgemäßen Zusammensetzungen enthalten weiterhin mindestens einen hydrophoben Coemulgator. Als hydrophober Coemulgator bevorzugt sind Öle und fettähnliche Substanzen, die einen HLB-Wert von 1 - 6, bevorzugt von 1 - 5,6, besonders bevorzugt 1,5 - 5,0 und außerordentlich bevorzugt Werte von 2,0 , 2,1 , 2,2 , 2,3 , 2,4 , 2,5 , 2,6, 2,7 , 2,8 , 2,9 , 3,0 aufweisen, die also sowohl als kosmetisches Emollient als auch emulgierend wirken. Der hydrophobe Coemulgator kann bei 20°C unter Normalbedingungen als Feststoff, Paste oder Flüssigkeit vorliegen. Für bestimmte Anwendungseigenschaften, insbesondere für das Gleitvermögen (lubricity), die optische Klarheit und/oder die gelartige Konsistenz der erfindungsgemäßen Zusammensetzungen, können die bei 20°C flüssigen hydrophoben Coemulgatoren bevorzugt sein. Besonders bevorzugte bei 20°C flüssige hydrophobe Coemulgatoren sind Propylenglycolmono- und -diester von linearen oder verzweigten, gesättigten oder ungesättigten C₆-C₂₂-Carbonsäuren, insbesondere Propylenglycolmonoisostearat, Propylenglycoldipelargonat, Propylenglycolmonooleat, Propylenglycolmonomyristat, sowie Mischungen hiervon, wobei Propylenglycolmonoisostearat außerordentlich bevorzugt ist.
Weitere erfindungsgemäß bevorzugte hydrophobe Coemulgatoren, die bei 20°C flüssig oder fest sein können, sind ausgewählt aus Glycerinmono- und -diestern von linearen oder verzweigten, gesättigten oder ungesättigten C₆-C₂₂-Carbonsäuren, insbesondere Glycerinmonooleat, Glycerinmonostearat, Glycerinmonopalmitat, Glycerinmonomyristat, Glycerinmonococoat, Glycerinmonoisostearat, Glycerindüsostearat, Glycerinmonolanolat und Glycerinmonolaurat sowie Mischungen hiervon, wobei die genannten Glycerinmonoester besonders bevorzugt sind. Außerordentlich bevorzugt ist Glycerinmonooleat.
Weitere erfindungsgemäß bevorzugte hydrophobe Coemulgatoren, die bei 20°C flüssig oder fest sein können, sind ausgewählt aus Sorbitanmono-, -di- und -triestern von linearen oder verzweigten, gesättigten oder ungesättigten C₆-C₂₂-Carbonsäuren, insbesondere den Sorbitanmonoestem wie Sorbitanmonopalmitat, Sorbitanmonooleat, Sorbitanmonostearat, Sorbitanmonomyristat, Sorbitanmonococoat und Sorbitanmonoisostearat.
Die erfindungsgemäßen Zusammensetzungen enthalten den/die hydrophoben Coemulgator/en bevorzugt insgesamt in Mengen von 0,5 - 7 Gew.-%, besonders bevorzugt 1,0 - 5,0 Gew.-% und außerordentlich bevorzugt 1,3 - 3,0 Gew.-%, jeweils bezogen auf das Gesamtgewicht der treibmittelfreien Zusammensetzung.

Die erfindungsgemäßen Zusammensetzungen enthalten weiterhin mindestens ein wasserlösliches C₃ - C₉-Polyol sowie Mischungen hiervon. Unter Wasserlöslichkeit versteht man erfindungsgemäß, dass sich wenigstens 5 Gew.-% des Polyols bei 20 °C in Wasser klar lösen oder aber - im Falle längerkettiger Polyole - durch Erwärmen der Lösung auf 50 °C bis 60 °C in Lösung gebracht werden können. Bevorzugt sind diese Komponenten ausgewählt aus 1,2-Propylenglycol, 2-Methyl-1,3-propandiol, Glycerin, Butandiolen wie 1,2-Butandiol, 1,3-Butandiol und 1,4-Butandiol, Pentandiolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Besonders bevorzugt sind 1,2-Propylenglycol, Glycerin und Sorbit. Auch Zucker und bestimmte Zuckerderivate wie Fructose, Glucose, Maltose, Maltitol, Mannit, Inosit, Sucrose, Trehalose und Xylose sind erfindungsgemäß geeignet. Die C₃ - C₉-Polyole dienen als Hautfeuchthaltemittel. Überraschend wurde festgestellt, dass ein Gehalt an C₃ - C₉-Polyolen die Zusammensetzung vor einem vorschnellen Austrocknen bewahrt und somit eine Verstopfung des Dispenser-Ventils vermieden wird.
Die erfindungsgemäßen Zusammensetzungen enthalten das mindestens eine wasserlösliche mehrwertige C₃ - C₉-Alkanol bevorzugt insgesamt in Mengen von 0,1 - 10 Gew.-%, besonders bevorzugt 0,5 - 5 Gew.-% und außerordentlich bevorzugt 1,0 - 2,0 Gew.-%, jeweils bezogen auf das Gesamtgewicht der treibmittelfreien Zusammensetzung.

Die erfindungsgemäßen Zusammensetzungen enthalten weiterhin mindestens ein verdickendes Polymer, das kein Polyethylenglycol der allgemeinen Formel H-(OCH₂CH₂)ₙ-OH darstellt. Erfindungsgemäß bevorzugt sind die organischen Verdickungsmittel, insbesondere die gewünschtenfalls chemisch modifizierten CelluloseDerivate, vorzugsweise Hydroxyalkylcellulosen wie Hydroxyethylcellulose, Hydroxypropylcellulose, Hydroxypropylmethylcellulose, Methylcellulose, Hydroxymethylcellulose und Carboxymethylcellulose, weiterhin insbesondere Stärken sowie Stärkeabbauprodukte wie Amylose und Amylopektin, chemisch und/oder thermisch modifizierte Stärken, z. B. Hydroxypropylstärkephosphat, Dihydroxypropyldistärkephosphat oder die Handelsprodukte Dry Flo^{®} , weiterhin Chitosan und dessen Derivate.
Polysaccharide, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack, Gelatine und Agar-Agar, können ebenfalls enthalten sein, sind aber weniger bevorzugt. In einer besonders bevorzugten Ausführungsform sind die erfindungsgemäßen Zusammensetzungen frei von Polysaccharid-Gums, insbesondere frei von Guar-Gum, Xanthan-Gum, Alginaten, Gummi arabicum, Karaya-Gummi, Carrageenanen, Johannisbrotkernmehl, Leinsamen-Gums, Schellack, Gelatine und/oder Agar-Agar.
Weitere erfindungsgemäß bevorzugte organische Verdickungsmittel sind Polyalkylenglycolether der allgemeinen Formel R-O-(CH₂-CH₂-O)ₙ-R' oder R-O-(CH₂-CH(CH₃)-O)ₙ-R' und Polyalkylenglycolester der allgemeinen Formel R-COO-(CH₂-CH₂-O)ₙ-C(O)-R' oder R-COO-(CH₂-CH(CH₃)-O)ₙ-C(O)-R', wobei R und R' unabhängig voneinander für eine lineare oder verzweigte C₆-C₃₀-Alkyl- oder C₆-C₃₀-Alkenylgruppe stehen und n, die durchschnittliche Anzahl der Alkylenoxid-Einheiten, für eine Zahl größer 75, bevorzugt für eine Zahl von 100 - 400 und besonders bevorzugt für eine Zahl von 120-150 steht. R und R' sind bevorzugt unabhängig voneinander eine Decyl-, Undecyl-, Lauryl, Tridecyl-, Tetradecyl-, Pentadecyl, Hexadecyl-, Heptadecyl-, Heptadecenyl-, Octadecyl-, Octadecenyl-, Stearyl-, Oleyl-, Nonadecyl-, Nonadecenyl-, Eicosyl-, Eicosenyl-, Docosyl-, Docosenyl- oder Behenyl-Gruppe. Erfindungsgemäß bevorzugt sind die Polyethylenglycolether und Polyethylenglycolester, besonders PEG-150-Distearat, PEG-150-Dioleat, PEG-120-Methylglucosedioleat, PEG-300-Pentaerythrityltetraisostearat, PEG-350-Sorbitantriisostearat und PEG-230-Glycerintriisostearat.
Weitere erfindungsgemäß bevorzugte organische Verdickungsmittel sind synthetische anionische Homo- und Copolymere aus Acrylsäure, Methacrylsäure, Crotonsäure, Maleinsäureanhydrid oder 2-Acrylamido-2-methylpropansulfonsäure, wobei die sauren Gruppen ganz oder teilweise als Natrium-, Kalium-, Ammonium-, Mono- oder TriethanolammoniumSalz vorliegen können. Die Copolymere können mindestens ein nichtionogenes Monomer enthalten. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS, Simulgel^{®} EG und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich). Weitere bevorzugte organische anionische Verdickungsmittel sind vernetzte Copolymere aus Vinylpyrrolidon und 2-Methyl-2[(1-oxo-2-propenyl)amino]-1-propansulfonsäure, die teilweise oder vollständig neutralisiert sind, wie sie z. B. unter dem Handelsnamen Aristoflex^{®} AVC erhältlich sind.
Weitere erfindungsgemäß bevorzugte organische Verdickungsmittel sind synthetische kationische Homo- und Copolymere. Bevorzugte kationische Homo- und Copolymere enthalten quartäre Ammoniumgruppen. Besonders bevorzugt sind Polymere, bei denen quartäre Ammoniumgruppen über eine C₁₋₄-Kohlenwasserstoffgruppe an eine aus Acrylsäure, Methacrylsäure oder deren Derivaten aufgebaute Polymerhauptkette gebunden sind.
Homopolymere der allgemeinen Formel (I), 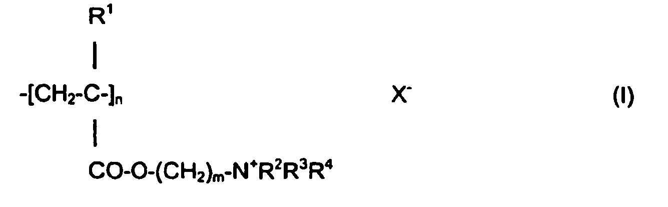 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.
Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.
Ein besonders geeignetes Homopolymer ist das, gewünschtenfalls vernetzte, Poly(methacryloyloxyethyltrimethylammoniumchlorid) mit der INCI-Bezeichnung Polyquaternium-37. Es wird bevorzugt in Form einer nichtwässrigen Polymerdispersion, die einen Polymeranteil nicht unter 30 Gew.-% aufweisen sollte, eingesetzt. Solche Polymerdispersionen sind unter den Handelsnamen Salcare^{®} SC 95 (ca. 50 % Polymeranteil, weitere Komponenten: Mineral Oil und PPG-1-Trideceth-6) und Salcare^{®} SC 96 (ca. 50 % Polymeranteil, weitere Komponenten: Propylene Glycol Dicaprylate/Dicaprate und PPG-1-Trideceth-6) erhältlich.
Copolymere mit Monomereinheiten gemäß Formel (I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind als ca. 50 %ige nichtwässrige Polymerdispersion unter dem Handelsnamen Salcare^{®} SC 92 erhältlich.
Weitere bevorzugte kationische Polymere sind beispielsweise polymere Dimethyldiallylammoniumsalze und deren Copolymere mit Estern und Amiden von Acrylsäure und Methacrylsäure, wie sie unter den Handelsnamen Merquat^{®} 100 (Poly(dimethyldiallylammoniumchlorid)) und Merquat^{®}550 bzw. Merquat^{®} S (Dimethyldiallylammoniumchlorid-Acrylamid-Copolymere, INCI-Bezeichnung: Polyquatemium-7) erhältlich sind.
Die gleichzeitige Anwesenheit von mindestens einem 2-Acrylamido-2-methylpropansulfonsäure-Polymer (anionisch) und mindestens einem Diallyldimethylallylammoniumchlorid-Acrylamid-Copolymer (kationisch) führt zu Inkompatibilitäten, die die Anwendungseigenschaften beeinträchtigen. Daher enthalten die erfindungsgemäßen Zusammensetzungen nicht gleichzeitig mindestens ein 2-Acrylamido-2-methylpropansulfonsäure-Polymer und mindestens ein Diallyldimethylallylammoniumchlorid-Acrylamid-Copolymer als verdickende Polymere.
Weitere erfindungsgemäß bevorzugte anorganische polymere Verdickungsmittel sind natürliche und synthetische Tone und Schichtsilikate und Aluminiumsilikate wie beispielsweise Bentonite, Hectorit, Montmorillonit oder Laponite^{®}.
Die erfindungsgemäßen Zusammensetzungen enthalten mindestens ein verdickendes Polymer, das kein Polyethylenglycol darstellt, bevorzugt insgesamt in Mengen von 0,01 - 3 Gew.-%, besonders bevorzugt 0,1 - 1 Gew.-% und außerordentlich bevorzugt 0,3 - 0,7 Gew.-%, jeweils bezogen auf das Gesamtgewicht der treibmittelfreien Zusammensetzung. Die oben erläuterten Verdickungsmittel Polyalkylenglycolether der allgemeinen Formel R-O-(CH₂-CH₂-O)ₙ-R' oder R-O-(CH₂-CH(CH₃)-O)ₙ-R' oder Polyalkylenglycolester der allgemeinen Formel R-COO-(CH₂-CH₂-O)ₙ-C(O)-R' oder R-COO-(CH₂-CH(CH₃)-O)ₙ-C(O)-R' sind bevorzugt in Gesamtmengen von 0,01 - 0,1 Gew.-%, besonders bevorzugt 0,02 - 0,04 Gew.-% und außerordentlich bevorzugt 0,025 - 0,035 Gew.-%, enthalten, jeweils bezogen auf das Gesamtgewicht der treibmittelfreien Zusammensetzung.

Die erfindungsgemäßen Zusammensetzungen enthalten weiterhin mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung. Dieses Polyethylenglycol mit einem mittleren Molekulargewicht von mindestens 300.000 Dalton weist die allgemeine Strukturformel H-(OCH₂CH₂)ₙ-OH auf.
Derartige Polyethylenglycole sind zum Beispiel ein Polyethylenglycol mit einem mittleren Molekulargewicht von 600.000 Dalton mit der INCI-Bezeichnung PEG-14M, erhältlich z. B. unter den Handelsnamenen Polyox WSR N-3000 oder Polyox WSR 205 von Amerchol, ein Polyethylenglycol mit einem mittleren Molekulargewicht von 1.000.000 Dalton mit der INCI-Bezeichnung PEG-23M, erhältlich z. B. unter dem Handelsnamen Polyox WSR N-12K von Amerchol, ein Polyethylenglycol mit einem mittleren Molekulargewicht von 2.000.000 Dalton mit der lNCl-Bezeichnung PEG-45M, erhältlich z. B. unter dem Handelsnamen Polyox WSR N-60K von Amerchol, ein Polyethylenglycol mit einem mittleren Molekulargewicht von 4.000.000 Dalton mit der INCI-Bezeichnung PEG-90M, erhältlich z. B. unter dem Handelsnamen Polyox WSR 301 von Amerchol, ein Polyethylenglycol mit einem mittleren Molekulargewicht von 5.000.000 Dalton mit der INCI-Bezeichnung PEG-115M, erhältlich z. B. unter dem Handelsnamen Polyox Coagulant von Amerchol.
Bevorzugt sind Polyethylenglycole mit einem mittleren Molekulargewicht von 600.000 - 7.000.000 Dalton, besonders bevorzugt sind Polyethylenglycole mit einem mittleren Molekulargewicht von 2.000.000 - 5.000.000 Dalton, außerordentlich bevorzugt ist ein Polyethylenglycol mit einem mittleren Molekulargewicht von 4.000.000 Dalton. Es kann erfindungsgemäß bevorzugt sein, Mischungen der genannten hochmolekularen Polyethylenglycole einzusetzen.
Das mindestens eine Polyethylenglycol mit einem mittleren Molekulargewicht von mindestens 300.000 Dalton ist einer Gesamtmenge von 0,001 - 0,08 Gew.-%, bevorzugt 0,005 - 0,03 Gew.-% und besonders bevorzugt 0,008 - 0,02 Gew.-%, jeweils bezogen auf die treibmittelfreie Zusammensetzung, enthalten. Besonders bevorzugt sind Konzentrationen von 0,009, 0,01 und 0,015 Gew.-%, jeweils bezogen auf die treibmittelfreie Zusammensetzung. Es war überraschend und für den Fachmann nicht vorauszusehen, dass mit derartig geringen Konzentrationen ein gutes Gleiten der Klinge auf der Haut und insgesamt ein hervorragendes Hautgefühl erzielt und gleichzeitig die Klebrigkeit der Zusammensetzung herabgesetzt und die Qualität des erzeugten Schaums verbessert werden konnte.

Erfindungsgemäß bevorzugte Zusammensetzungen sind dadurch gekennzeichnet, dass sie weiterhin wenigstens ein Polyalkylenglycol-modifiziertes Silicon enthalten. Polyalkylenglycol-modifizierte Silicone ermöglichen die Formulierung hochtransparenter Zusammensetzungen, reduzieren die Klebrigkeit und hinterlassen ein frisches Hautgefühl. Unter Polyalkylenglycol-modifizierten Siliconen werden erfindungsgemäß Polyorganosiloxane mit Polyethylenglycol-, Polypropylenglycol- oder Polyethylenglycol/Polypropylenglycol-End- oder Seitenketten verstanden. Erfindungsgemäß besonders bevorzugt sind sogenannte Silicon-Copolyole, insbesondere solche Verbindungen mit der früheren INCI-Bezeichnung Dimethicone Copolyol, die beispielsweise von Wacker-Chemie unter der Bezeichnung Belsil^{®} DMC 6031, Belsil^{®} DMC 6032, Belsil^{®} DMC 6038 oder **Belsil^{®}** DMC 3071 VP bzw. von Dow Corning unter der Bezeichnung DC 2501 bzw. von Degussa unter der Bezeichnung Abil EM 97 im Handel sind. Erfindungsgemäß kann auch ein beliebiges Gemisch dieser Silicone eingesetzt werden. Weitere hydrophil modifizierte Silicon-Copolyole sind ausgewählt aus den Poly-(C₂-C₃)-alkylenglycol-modifizierten Siliconen, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11-12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3-30 und besonders bevorzugt 12-20, insbesondere 14 - 18, stehen), Bis-PEG/PPG-c/d Dimethicone (wobei c und d unabhängig voneinander für Zahlen von 10-25, bevorzugt 14-20 und besonders bevorzugt 14-16. stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Beispiele für derartige Silicone sind PEG/PPG-18/18 Dimethicone, das in einer 1:9-Mischung mit Cyclomethicone unter der Bezeichnung DC 3225 C bzw. DC 5225 C von Dow Corning im Handel erhältlich ist, sowie Bis-PEG/PPG-14/14 Dimethicone, das in einer Mischung mit Cyclomethicone unter der Bezeichnung Abil EM 97 (Goldschmidt) im Handel erhältlich ist. Erfindungsgemäß besonders geeignet sind PEG-x Dimethicone mit x = 2 - 20, bevorzugt x = 3 - 17, besonders bevorzugt x = 11 - 12, außerordentlich bevorzugt ist das Handelsprodukt Dow Corning 193 Surfactant mit der INCI-Bezeichnung PEG-12 Dimethicone.

Weiterhin geeignet sind Poly-(C₂-C₃)alkylenglycol-modifizierte Silicone, die mit C₄-C₁₈₋Alkylgruppen hydrophob modifiziert sind, besonders bevorzugt Cetyl PEG/PPG-10/1 Dimethicone (früher: Cetyl Dimethicone Copolyol, erhältlich unter der Bezeichnung Abil EM 90), weiterhin Alkyl Methicone Copolyole und Alkyl Dimethicone Ethoxy Glucoside.
Die Gesamtmenge des/der Polyalkylenglycol-modifizierten Silicons/Silicone in den erfindungsgemäßen Zusammensetzungen beträgt bevorzugt 0,1-8 Gew.-%, besonders bevorzugt 0,5 - 5 Gew.-% und außerordentlich bevorzugt 1 - 3 Gew.-%, jeweils bezogen auf das Gesamtgewicht der treibmittelfreien Zusammensetzung.

In einer weiteren bevorzugten Ausführungsform können die erfindungsgemäßen Zusammensetzungen zur Verbesserung ihrer hautpflegenden Wirkung mindestens ein Öl und/oder mindestens einen Fettstoff enthalten, das/der nicht zur Gruppe der hydrophoben Coemulgatoren gehört, da es/er keine emulgierenden Eigenschaften aufweist. Erfindungsgemäß bevorzugte Öle und Fettstoffe sind ausgewählt aus
- flüssigen Paraffinölen, Isoparaffinölen und synthetischen Kohlenwasserstoffen, z. B. 1,3-Di-(2-ethyl-hexyl)-cyclohexan (Cetiol^{®} S), Polydecen oder Polyisobuten,
- Di-n-alkylethern mit insgesamt 12 bis 36, insbesondere 12 bis 24 C-Atomen, z. B. Din-octylether (Cetiol^{®} OE), n-Hexyl-n-octylether und n-Octyl-n-decylether,
- pflanzlichen Ölen, wie Avocadoöl, Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls,
- Esterölen und Wachsestern, das heißt, Estern von C₆₋₃₀-Mono- oder Dicarbonsäuren mit linearen oder verzweigten C₂₋₃₀-Alkanolen. Erfindungsgemäß besonders bevorzugt sind Isopropylmyristat, Isopropylpalmitat, Isopropylisostearat, 2-Ethylhexylpalmitat, Stearinsäure-2-ethylhexylester, Cetyloleat, Glycerintricaprylat, Kokosfettalkoholcaprinat/-caprylat, n-Butylstearat, Oleylerucat, Oleyloleat, Hexyllaurat, Di-n-butyladipat, Di-isopropyladipat, Di-(2-ethylhexyl)-adipat, Di-(2-ethylhexyl)-succinat, Di-isotridecylacelaat, Myristylmyristat, Cetearylisononanoat und Ölsäuredecylester;
- Hydroxycarbonsäurealkylestern, wobei die Vollester der Glycolsäure, Milchsäure, Äpfelsäure, Weinsäure oder Citronensäure bevorzugt sind, aber auch Ester der β-Hydroxypropionsäure, der Tartronsäure, der D-Gluconsäure, Zuckersäure, Schleimsäure oder Glucuronsäure geeignet sind und besonders bevorzugt die Ester von C₁₂₋C₁₅-Fettalkoholen, z. B. die Handelsprodukte Cosmacol^{®} der EniChem, Augusta Industriale, sind,
- symmetrischen, unsymmetrischen oder cyclischen Estern der Kohlensäure mit Fettalkoholen, z. B. Glycerincarbonat oder Dicaprylylcarbonat (Cetiol^{®} CC),
- Siliconverbindungen, bevorzugt ausgewählt aus Decamethylcyclopentasiloxan, Dodecamethylcyclohexasiloxan und linearen Siliconpolymeren, die gewünschtenfalls quervernetzt sein können, z. B. Polydialkylsiloxanen wie bevorzugt Hexamethyldisiloxan (L₂), Octamethyltrisiloxan (L₃), 3-Hexylheptamethyltrisiloxan (z. B. Dow Corning 2-1731), Decamethyltetrasiloxan (L₄), Dodecamethylpentasiloxan (L₅), Polyalkylarylsiloxanen, zum Beispiel Poly(methylphenylsiloxan), sowie Mischungen der vorgenannten Substanzen, insbesondere bevorzugt Mischungen aus L₃ und L₄, wie sie unter der Bezeichnung Dow Corning 2-1184 erhältlich sind, weiterhin bevorzugt die unter der Bezeichnung Dow Corning 200 Fluid erhältlichen Polymere mit unterschiedlicher Kettenlänge und Viskosität (INCI-Bezeichnung: Dimethicone).

Besonders bevorzugte Öle sind flüssige Paraffinöle, Isoparaffinöle und synthetische Kohlenwasserstoffe, außerordentlich bevorzugt sind Paraffinöle.
Bevorzugte erfindungsgemäße Zusammensetzungen sind dadurch gekennzeichnet, dass sie mindestens ein Öl und/oder mindestens einen Fettstoff, das oder der nicht zur Gruppe der hydrophoben Coemulgatoren gehört, bevorzugt mindestens ein Paraffinöl, oder Mischungen hiervon insgesamt in Mengen von 0,1-4 Gew.-%, besonders bevorzugt 0,5 - 3,5 Gew.-% und außerordentlich bevorzugt 1 - 3 Gew.-%, jeweils bezogen auf das Gesamtgewicht der treibmittelfreien Zusammensetzung, enthalten.

Weiterhin wurde überraschend festgestellt, dass durch einen Gehalt an mindestens einer Kieselsäure die rheologischen und/oder sensorischen Eigenschaften der erfindungsgemäßen Zusammensetzungen in für den Fachmann nicht zu erwartendem Ausmaß verbessert werden konnten. Die erfindungsgemäßen Zusammensetzungen enthalten daher zur Verbesserung der rheologischen und/oder sensorischen Eigenschaften mindestens eine Kieselsäure. Die Kieselsäure ist ausgewählt aus pyrogenen und Fällungskieselsäuren sowie Mischungen hiervon, wobei Fällungskieselsäuren erfindungsgemäß besonders bevorzugt sind.
Besonders bevorzugte erfindungsgemäße Zusammensetzungen sind dadurch gekennzeichnet, dass sie Kieselsäure insgesamt in Mengen von 0,01 - 1 Gew.-%, bevorzugt 0,1 - 0,7 Gew.-% und besonders bevorzugt 0,2 - 0,5 Gew.-%, jeweils bezogen auf das Gesamtgewicht der treibmittelfreien Zusammensetzung, enthalten.

In einer weiteren bevorzugten Ausführungsform können die erfindungsgemäßen Zusammensetzungen zur Verbesserung ihrer hauterfrischenden Wirkung mindestens einen hautkühlenden Wirkstoff enthalten. Erfindungsgemäß geeignete hautkühlende Wirkstoffe sind beispielsweise ausgewählt aus Menthol, Isopulegol sowie Mentholderivaten, z. B. Menthyllactat, Menthylpyrrolidoncarbonsäure, Menthylmethylether, Menthoxypropandiol, Mentholethylenglycolcarbonat, Mentholpropylenglycolcarbonat, Menthonglycerinacetal (9-Methyl-6-(1-methylethyl)-1,4-dioxaspiro(4.5)decan-2-methanol), Monomenthylsuccinat und 2- Hydroxymethyl-3,5,5-trimethylcyclohexanol. Besonders bevorzugt sind Menthol, Isopulegol, Menthyllactat, Mentholethylenglycolcarbonat, Mentholpropylenglycolcarbonat, Menthoxypropandiol und Menthylpyrrolidoncarbonsäure sowie Mischungen dieser Substanzen.
Besonders bevorzugte erfindungsgemäße Zusammensetzungen sind dadurch gekennzeichnet, dass sie mindestens einen hautkühlenden Wirkstoff in Mengen von insgesamt 0,01 - 1 Gew.%, bevorzugt 0,02 - 0,5 Gew.% und besonders bevorzugt 0,1 - 0,3 Gew.%, jeweils bezogen auf das Gesamtgewicht der treibmittelfreien Zusammensetzung, enthalten.

Die erfindungsgemäßen kosmetischen Zusammensetzungen können bevorzugt aus Zweikammeraerosolbehältem entnommen und auf die Haut aufgetragen werden. Erfindungsgemäß bevorzugte Packmittel sind Behältnisse, in der sich eine Kammer mit einer Füllung aus den flüssigen bzw. breiartigen Stoffen unter dem Druck eines in einer zweiten Kammer befindlichen stehenden Primärtreibmittels befindet. Derartige Behälter können mit Ventilen sehr unterschiedlicher Bauart ausgestattet sein, die die Entnahme des Inhalts der ersten Kammer als Emulsion oder Gel in jeder Lage - auch mit einem unten angeordneten Ventil oder aus einem auf dem Kopf stehenden Behälter - ermöglichen. In einer besonders bevorzugten Ausführungsform sind die erfindungsgemäßen Zusammensetzungen in einem Aerosolbehälter, bei dem das Produkt in einem flexiblen Beutel aus Metall oder Kunststoff innerhalb der Dose eingeschlossen ist, verpackt. Derartige Behälter sind unter dem Handelsnamen BiCan® erhältlich.
Erfindungsgemäße Zusammensetzungen stellen ungeschäumt, also unmittelbar nach dem Austreten aus dem Aerosolbehälter, Zwei- oder Mehrphasensysteme - in der Regel Emulsionen - dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.
Es hat sich darüber hinaus in überraschender Weise herausgestellt, dass bei der Verwendung von Treibmitteln, besonders vorteilhaft von in der gegebenenfalls vorhandenen Ölphase löslichen Treibmitteln, also beispielsweise Propan, Butan, Isobutan oder üblichen Propan-Butan- bzw. Propanilsobutan-Gemischen, die erfindungsgemäßen Zusammensetzungen nicht einfach als Aerosoltröpfchen versprüht werden, sondern sich zu feinblasigen, reichhaltigen Schäumen entwickeln, sobald die mit solchen Treibmitteln beladenen Systeme Druckentspannung erfahren.
Bei Verwendung von gesättigten aliphatischen Kohlenwasserstoffen mit 5 oder 6 Kohlenstoffatomen, wie n-Pentan, Isopentan, Neopentan und Hexan, bevorzugt n-Pentan, Isopentan und deren Gemischen, sogenannten Sekundärtreibmitteln, kann man das selbständige Aufschäumen nach dem Austritt aus der Druckverpackung zeitlich verzögern und ein sogenanntes Nachschäumen erzielen. Besonders bevorzugt sind Primär- und Sekundärtreibmittel-Mischungen aus Isopentan und Isobutan im Gewichtsverhältnis Isopentan/Isobutan von 1:1 bis 9:1, außerordentlich bevorzugt 2:1 bis 7:1, und am bevorzugtesten 7:3 bis 3:1.
Durch das Verdampfen des Sekundärtreibmittels im applizierten Kosmetikprodukt wird der Haut Wärme entzogen und ein angenehmer Kühleffekt erzielt.
Die Primär- und Sekundärtreibmittel-Mischung wird bevorzugt so gewählt, dass sie bei 20°C einen Dampfdruck von 34-140 kPa, bevorzugt 34-105 kPa, erzeugt. Die Primär- und Sekundärtreibmittel-Mischung wird erfindungsgemäß so gewählt, dass die kosmetische Zusammensetzung nach dem Austritt aus dem Dispenser beim Kontakt mit der Haut genügend schnell aufschäumt, typischerweise in 2 - 40 Sekunden, bevorzugt in 5-30 Sekunden.
Das Gewichtsverhältnis zwischen Treibmittel und treibmittelfreier Seifenzusammensetzung beträgt 20 : 80 - 1 : 99, bevorzugt 10 : 90 - 2 : 98 und besonders bevorzugt 5 : 95 - 3 : 97. Es können aber auch größere Mengen an Treibmittel in Bezug auf die treibmittelfreie Seifenzusammensetzung eingesetzt werden, z. B. in Gewichtsverhältnissen von 60: 40-30:70.

Ein weiterer Gegenstand der vorliegenden Erfindung ist ein nicht-therapeutisches, kosmetisches Verfahren zur Rasur und/oder zur Reinigung der Haut und/oder des Haars, bei dem eine kosmetische Zusammensetzung, die mit einem nachschäumenden Treibmittel aufgeschäumt werden kann und die Wasser, mindestens eine C₁₀-C₂₄-Alkancarbonsäure, mindestens ein Neutralisierungsmittel für die Alkancarbonsäure/n, mindestens ein nichtionisches Tensid, mindestens einen hydrophoben Coemulgator, mindestens ein wasserlösliches C₃-C₉-Polyol, mindestens ein verdickendes Polymer, das kein Polyethylenglykol darstellt, wobei nicht gleichzeitig mindestens ein 2-Acrylamido-2-methylpropansulfonsäure-Polymer und mindestens ein Diallyldimethylallylammoniumchlorid-Acrylamid-Copolymer enthalten sind, mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung, und weiterhin Kieselsäure enthält, auf die Haut und/oder die Haare aufgetragen und anschließend gewünschtenfalls abgespült und/oder abgewischt wird. Das Entfernen der Zusammensetzung kann nach einer Einwirkzeit von ca. 5 Sekunden bis 20 Minuten oder länger erfolgen. Für die Rasur bevorzugt ist eine Einwirkzeit von 10 bis 90 Sekunden.

Besonders bevorzugte erfindungsgemäße Zusammensetzungen sind dadurch gekennzeichnet, dass sie mindestens einen hautfeuchthaltenden Wirkstoff enthalten. Besonders bevorzugte hautfeuchthaltende Wirkstoffe sind ausgewählt aus Aloe vera, Aminosäuren, deren Salzen und Estern, insbesondere Chitosoniumpyrrolidoncarbonsäuresalze, Proteinen, Proteinhydrolysaten Pyrrolidoncarbonsäure, weiteren Bestandteilen des "Natural Moisturizing Factor", der aus 40% freien Aminosäuren, 12% Pyrrolidoncarbonsäure, 12% Lactaten, 7% Harnstoff, 1,5% Harnsäure sowie D-Glucosamin, Kreatinin und verschiedenen Salzen besteht, Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen.
Erfindungsgemäß besonders bevorzugte Zusammensetzungen sind dadurch gekennzeichnet, dass sie mindestens einen hautfeuchthaltenden Wirkstoff in einer Gesamtmenge von 0,001 bis 10 Gew.-%, besonders bevorzugt 0,01 bis 5 Gew.-% und außerordentlich bevorzugt 0,1 bis 1 oder 2 Gew.-%, jeweils bezogen auf die gesamte treibmittelfreie Zusammensetzung, enthalten.

Erfindungsgemäß besonders bevorzugte Zusammensetzungen sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Panthenol, Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Erfindungsgemäß besonders bevorzugte Zusammensetzungen sind dadurch gekennzeichnet, dass sie mindestens einen hautberuhigenden Wirkstoff in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte treibmittelfreie Zusammensetzung, enthalten.

Ein weiterer Gegenstand der vorliegenden Erfindung ist die nicht-therapeutische Verwendung einer kosmetischen Zusammensetzung, die mit einem nachschäumenden Treibmittel aufgeschäumt werden kann und die Wasser, mindestens eine C₁₀- C₂₄-Alkancarbonsäure, mindestens ein Neutralisierungsmittel für die Alkancarbonsäure/n, mindestens ein nichtionisches Tensid, mindestens einen hydrophoben Coemulgator, mindestens ein wasserlösliches **C₃** - C₉-Polyol, mindestens ein verdickendes Polymer, das kein Polyethylenglycol darstellt, wobei nicht gleichzeitig mindestens ein 2-Acrylamido-2-methylpropansulfonsäure-Polymer und mindestens ein Diallyldimethylallylammoniumchlorid-Acrylamid-Copolymer enthalten ist, mindestens ein Polyethylenglycol mit einem mittleren Molekular gewicht von mindestens 300.000 Dalton in einer Menge von 0,001 - 0,08 Gew.-%, bezogen auf die treibmittelfreie Zusammensetzung, und weiterhin Kieselsäure enthält, zur Befeuchtung und/oder als Moisturizer für die Haut und/oder das Haar.

Ein weiterer Gegenstand der vorliegenden Erfindung ist die nicht-therapeutische Verwendung einer kosmetischen Zusammensetzung, die mit einem nachschäumenden Treibmittel aufgeschäumt werden kann und die Wasser, mindestens eine C₁₀ - C₂₄-Alkancarbonsäure, mindestens ein Neutralisierungsmittel für die Alkancarbonsäure/n, mindestens ein nichtionisches Tensid, mindestens einen hydrophoben Coemulgator, mindestens ein wasserlösliches C₃ - C₉-Polyol, mindestens ein verdickendes Polymer, das kein Polyethylenglycol darstellt, wobei nicht gleichzeitig mindestens ein 2-Acrylamido-2-methylpropansulfonsäure-Polymer und mindestens ein Diallyldimethylallylammonlumchlorid-Acrylamid-Copolymer enthalten ist, mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung, und weiterhin Kieselsäure enthält, zur Erfrischung für die Haut und/oder das Haar.

Ein weiterer Gegenstand der vorliegenden Erfindung ist die nicht-therapeutische Verwendung einer kosmetischen Zusammensetzung, die mit einem nachschäumenden Treibmittel aufgeschäumt werden kann und die Wasser, mindestens eine C₁₀-C₂₄-Alkancarbonsäure, mindestens ein Neutralisierungsmittel für die Alkancarbonsäure/n, mindestens ein nichtionisches Tensid, mindestens einen hydrophoben Coemulgator, mindestens ein wasserlösliches C₃-C₉-Polyol, mindestens ein verdickendes Polymer, das kein Polyethylenglycol darstellt, wobei nicht gleichzeitig mindestens ein 2-Acrylamido-2-methylpropansulfonsäure-Polymer und mindestens ein Diallyldimethylallylammoniumchlorid-Acrylamid-Copolymer enthalten ist, mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung, und weiterhin Kieselsäure enthält, als Rasierhilfsmittel und/oder Reinigungsmittel für die Haut und/oder das Haar.

Die erfindungsgemäßen Zusammensetzungen können ferner gegebenenfalls in der Kosmetik übliche Zusatzstoffe, beispielsweise Parfüms, Deodorantwirkstoffe, antimikrobielle Stoffe, Komplexierungs- und Sequestrierungsmittel, Antioxidantien, Perlglanzmittel, Pflanzenextrakte, insbesondere adstringierende Pflanzenextrakte, andere adstringierende und/oder porenverfeinernde und/oder porenverkleinernde Wirkstoffe, z. B. Aluminiumsalze, weiterhin Vitamine und Vitaminderivate, bevorzugt Retinylpalmitat, Niacinamid oder Tocopherylacetat, Mineralwässer, mineralische Salze, Konservierungsmittel, Bakterizide, Schaumstabilisatoren, organische Lösungsmittel, insbesondere Ethanol, Farbstoffe und/oder Pigmente enthalten.

Die nachfolgenden Beispiele Nr. 36, 39, 41 und 43 sollen die vorliegende Erfindung verdeutlichen, ohne sie hierauf einzuschränken. Die übrigen Beispiele sind nicht erfindungsgemäß. Alle Mengenangaben sind in Gew.-%, soweit nicht anders angegeben.

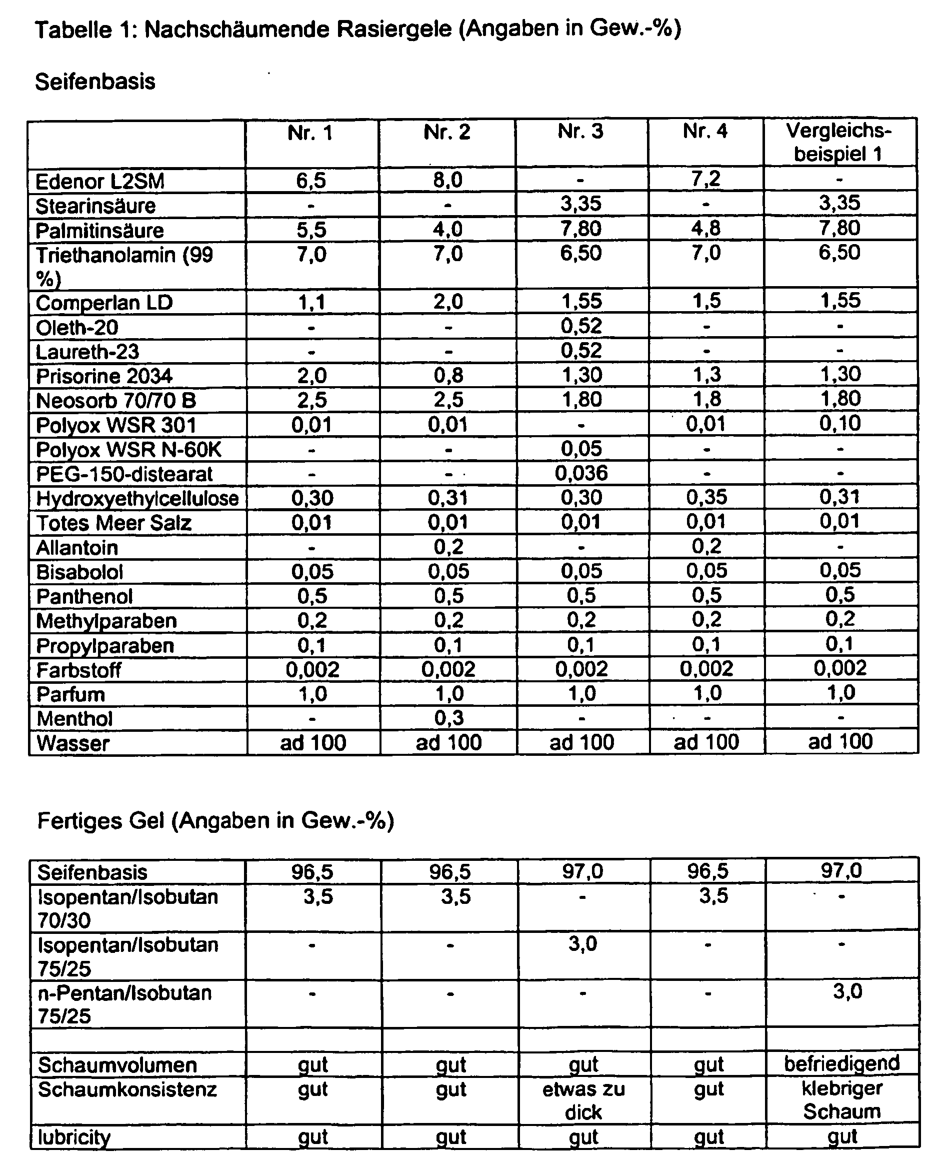

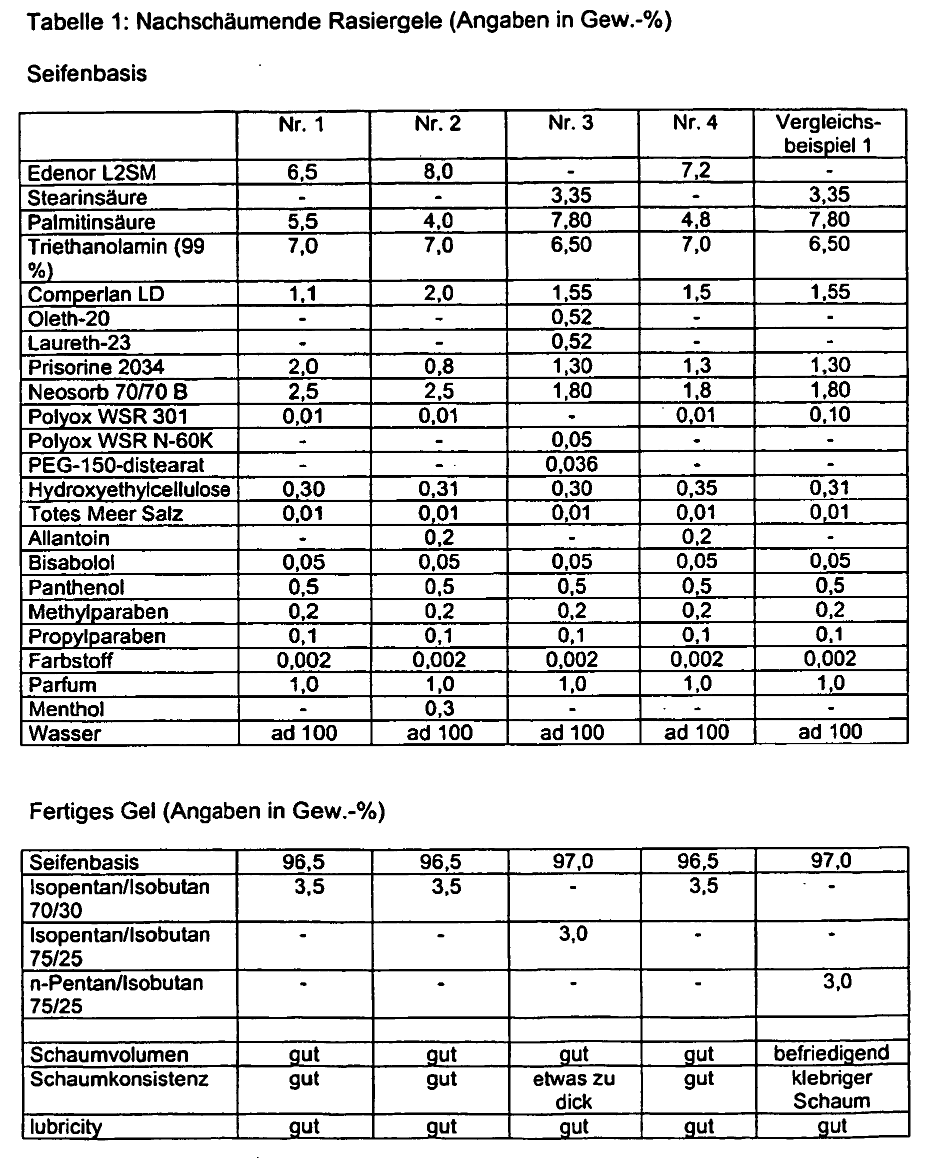

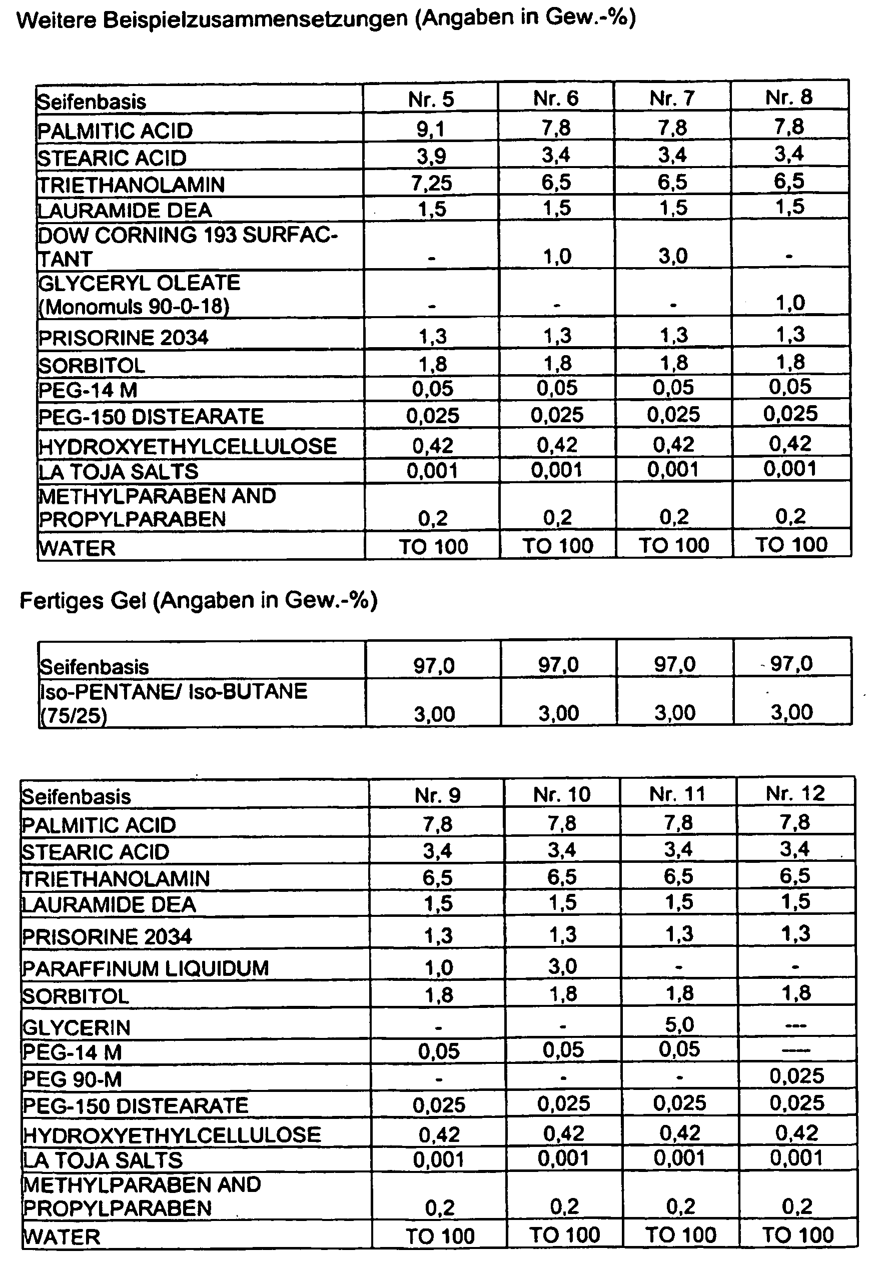

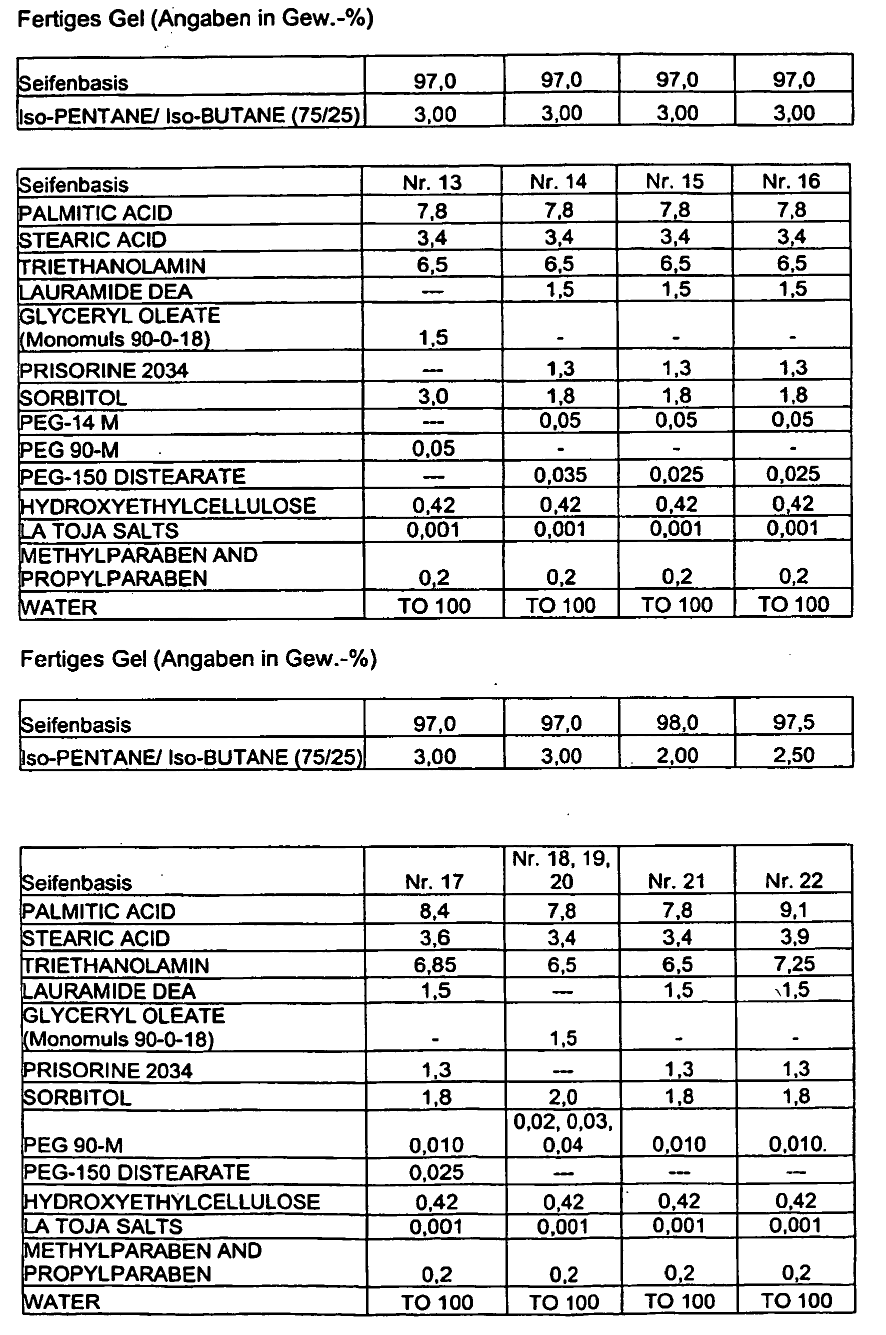

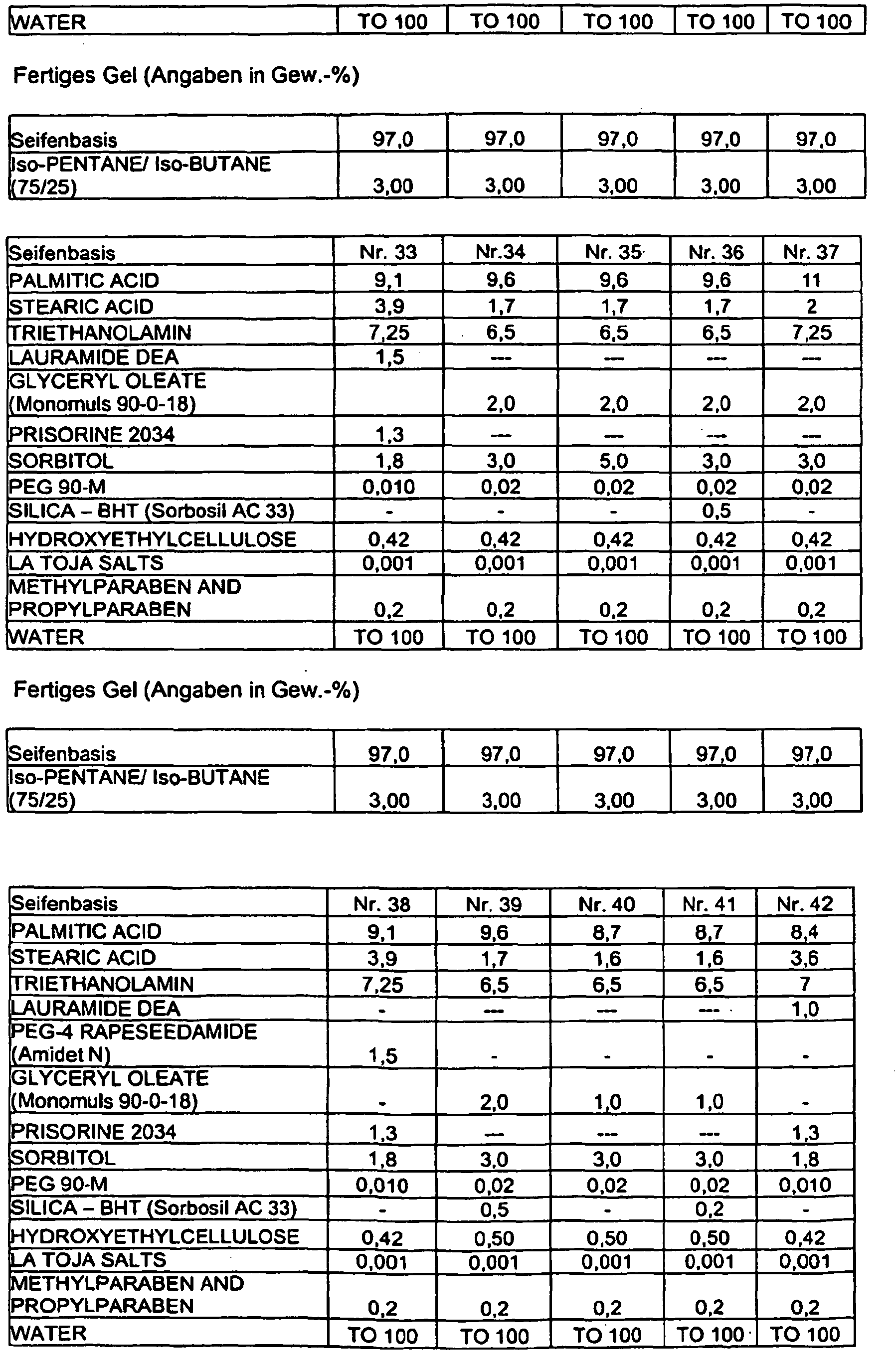

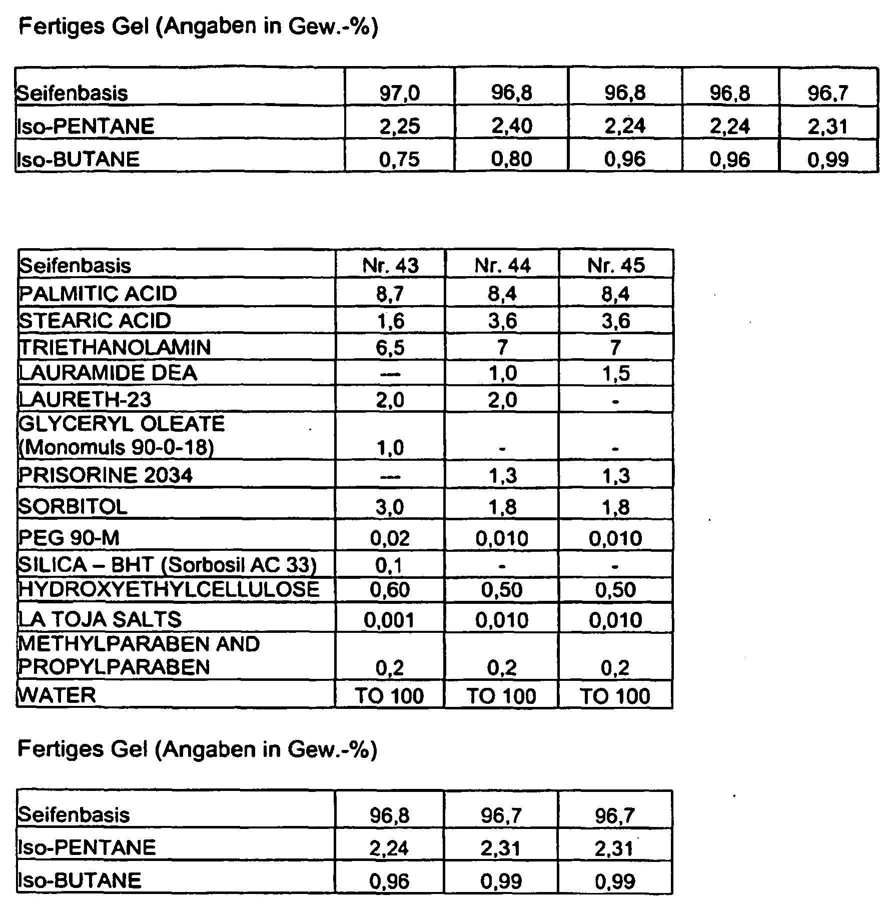

Die Gele 1 - 45 wurden zusammen mit dem Treibgas in einem Zweikammeraerosolbehälter Bican abgefüllt. Aus diesem Behälter wurde eine etwa haselnussgroße Menge entnommen und auf der Gesichtshaut verteilt. Dabei wurden Schaumvolumen und -konsistenz beurteilt. Beim anschließenden Rasieren wurde die "lubricity" beurteilt.

**Liste der verwendeten Rohstoffe**

| | | |
|---|---|---|
| Comperlan LD | Lauramide DEA (90 - 97 %) | Cognis |
| Dow Corning 193 Surfactant | PEG-12 Dimethicone | Dow Corning |
| Edenor L2SM | Palmitinsäure (40-55 %) und Stearinsäure (40-55 %) | Cognis |
| Neosorb 70/70 B | Sorbitol (70 % in Wasser) | Roquette |
| Polyox WSR 301 | PEG-90 M | Amerchol |
| Polyox WSR N-60K | PEG-45 M | Amerchol |
| Prisorine 2034 | Propylene Glycol Isostearate | Uniqema |
| Sorbosil AC 33 | Hydrate Silica | Ineos Silicas (Crossfield) |

## Patentansprüche

1. Kosmetische Zusammensetzung, die mit einem nachschäumenden Treibmittel aufgeschäumt werden kann, enthaltend
a) Wasser,
b) mindestens eine C₁₀ - C₂₄-Alkancarbonsäure,
c) mindestens ein Neutralisierungsmittel für b),
d) mindestens ein nichtionisches Tensid,
e) mindestens einen hydrophoben Coemulgator,
f) mindestens ein wasserlösliches C₃ - C₉-Polyol,
g) mindestens ein verdickendes Polymer, das kein Polyethylenglycol darstellt, wobei nicht gleichzeitig mindestens ein 2-Acrylamido-2-methylpropansulfonsäure-Polymer und mindestens ein Diallyldimethylallylammoniumchlorid-Acrylamid-Copolymer enthalten sind,
h) mindestens ein Polyethylenglycol mit einem mittleren Molekulargewicht von mindestens 300.000 Dalton in einer Menge von 0,001 - 0,08 Gew.-%, bezogen auf die treibmittelfreie Zusammensetzung,
**dadurch gekennzeichnet, dass**
i) Kieselsäure enthalten ist.

2. Kosmetische Zusammensetzung gemäß Anspruch 1, **dadurch gekennzeichnet, dass** die Kieselsäure aus Fällungskieselsäuren ausgewählt ist.

3. Kosmetische Zusammensetzung gemäß Anspruch 1 oder 2, **dadurch gekennzeichnet, dass** Kieselsäure insgesamt in Mengen von 0,01 - 1 Gew.-%, bevorzugt 0,1 - 0,7 Gew.-% und besonders bevorzugt 0,2 - 0,5 Gew.-%, jeweils bezogen auf das Gesamtgewicht der treibmittelfreien Zusammensetzung, enthalten ist.

4. Kosmetische Zusammensetzung gemäß einem der vorstehenden Ansprüche, **dadurch gekennzeichnet, dass** mindestens ein Polyethylenglycol mit einem mittleren Molekulargewicht von mindestens 300.000 Dalton in einer Menge von 0,005 - 0,03 Gew.-%, bezogen auf die treibmittelfreie Zusammensetzung, enthalten ist.

5. Kosmetische Zusammensetzung gemäß einem der vorstehenden Ansprüche, **dadurch gekennzeichnet, dass** das Polyethylenglycol ein mittleres Molekulargewicht von 600.000 Dalton bis 7 Millionen Dalton aufweist.

6. Kosmetische Zusammensetzung gemäß einem der vorstehenden Ansprüche, **dadurch gekennzeichnet, dass** das nichtionische Tensid ausgewählt ist aus Monoethanol- und Diethanolamiden von linearen C₆ - C₂₂-Alkancarbonsäuren und deren Mischungen, Anlagerungsprodukten von 1-10 Mol Ethylenoxid an die Amide von linearen C₆ - C₂₂-Alkancarbonsäuren und deren Mischungen sowie Anlagerungsprodukten von 2 bis 50 Mol Ethylenoxid an lineare und verzweigte, gesättigte und ungesättigte C₈ - C₃₀-Alkanole und C₈- C₃₀-Alkenole.

7. Kosmetische Zusammensetzung gemäß einem der vorstehenden Ansprüche, **dadurch gekennzeichnet, dass** die hydrophoben Coemulgatoren ausgewählt sind aus den Propylenglycolmono- und -diestern von linearen oder verzweigten, gesättigten oder ungesättigten C₆-C₂₂-Carbonsäuren, den Glycerinmono- und - diestern von linearen oder verzweigten, gesättigten oder ungesättigten C₆-C₂₂-Carbonsäuren und den Sorbitanmono-, -di- und -triestem von linearen oder verzweigten, gesättigten oder ungesättigten C₈-C₂₂-Carbonsäuren, die einen HLB-Wert von 1-6 aufweisen.

8. Kosmetische Zusammensetzung gemäß einem der vorstehenden Ansprüche, **dadurch gekennzeichnet, dass** das verdickende Polymer g) ausgewählt ist aus gewünschtenfalls chemisch modifizierten Cellulose-Derivaten, Stärken, Stärkeabbauprodukten, chemisch und/oder thermisch modifizierten Stärken, Chitosan und dessen Derivaten, Polyalkylenglycolethern der allgemeinen Formel R-O-(CH₂-CH₂-O)ₙ-R' oder R-O-(CH₂-CH(CH₃)-O)ₙ-R' und Polyalkylenglycolestern der allgemeinen Formel R-COO-(CH₂-CH₂-O)ₙ-C(O)-R' oder R-COO-(CH₂-CH(CH₃)-O)₆-C(O)-R', wobei R und R' unabhängig voneinander für eine lineare oder verzweigte C₈-C₃-Alkyl- oder C₆-C₃₀-Alkenylgruppe stehen und n für eine Zahl größer 75 steht, synthetischen anionischen Homo- und Copolymeren aus Acrylsäure, Methacrylsäure, Crotonsäure, Maleinsäureanhydrid oder 2-Acrylamido-2-methylpropansulfonsäure, synthetischen kationischen Homo- und Copolymeren, bei denen quartäre Ammoniumgruppen über eine C₁₋₄-Kohlenwasserstoffgruppe an eine aus Acrylsäure, Methacrylsäure oder deren Derivaten aufgebaute Polymerhauptkette gebunden sind, anorganischen polymeren Verdickungsmitteln sowie Mischungen dieser Polymere, wobei nicht gleichzeitig mindestens ein 2-Acrylamido-2-methylpropansulfonsäure-Polymer und mindestens ein Diallyldimethylallylammoniumchlorid-Acrylamid-Copolymer enthalten sind.

9. Kosmetische Zusammensetzung gemäß einem der vorstehenden Ansprüche, **dadurch gekennzeichnet, dass** sie frei von Polysaccharid-Gums ist.

10. Kosmetische Zusammensetzung gemäß einem der vorstehenden Ansprüche, **dadurch gekennzeichnet, dass** sie mindestens einen Polyalkylenglycolether der allgemeinen Formel R-O-(CH₂-CH₂-O)ₙ-R' oder R-O-(CH₂-CH(CH₃)-O)ₙ-R' und/oder mindestens einen Polyalkylenglycolester der allgemeinen Formel R-COO-(CH₂-CH₂-O)ₙ-**C(O)-R'** oder R-COO-(CH₂-CH(CH₃)-O)ₙ-C(O)-R', wobei R und R' unabhängig voneinander für eine lineare oder verzweigte C₆-C₃₀-Alkyl- oder C₈-C₃₀-Alkenylgruppe stehen und n, die durchschnittliche Anzahl der Alkylenoxid-Einheiten, für eine Zahl größer 75 steht, in Gesamtmengen von 0,01 - 0,1 Gew.-%, enthält, jeweils bezogen auf das Gesamtgewicht der treibmittelfreien Zusammensetzung.

11. Kosmetische Zusammensetzung gemäß einem der vorstehenden Ansprüche, **dadurch gekennzeichnet, dass** mindestens ein Polyalkylenglycol-modifiziertes Silicon enthalten ist.

12. Kosmetische Zusammensetzung gemäß einem der vorstehenden Ansprüche, **dadurch gekennzeichnet, dass** mindestens ein Öl und/oder mindestens ein Fettstoff oder Mischungen hiervon enthalten ist/sind.

13. Kosmetische Zusammensetzung gemäß einem der vorstehenden Ansprüche, **dadurch gekennzeichnet, dass** mindestens ein hautkühlender Wirkstoff enthalten ist.

14. Kosmetische Zusammensetzung gemäß einem der vorstehenden Ansprüche, **dadurch gekennzeichnet, dass** sie eine Treibmittelmischung aus Isopentan und Isobutan im Gewichtsverhältnis Isopentan/ Isobutan von 1:1 bis 9:1 enthält.

15. Kosmetische Zusammensetzung gemäß einem der vorstehenden Ansprüche, **dadurch gekennzeichnet, dass** das Gewichtsverhältnis zwischen Treibmittel und treibmittelfreier Seifenzusammensetzung 20:80-1 : 99 beträgt.

16. Nicht-therapeutische Verwendung einer kosmetischen Zusammensetzung gemäß einem der Ansprüche 1 - 15 als Rasierhilfsmittel oder als Reinigungsmittel für die

17. Nicht-therapeutische Verwendung einer kosmetischen Zusammensetzung gemäß einem der Ansprüche 1 - 15 zur Befeuchtung und/oder als Moisturizer und/oder zur Erfrischung

18. Nicht-therapeuusches, kosmetisches Verfahren zur Rasur und/oder zur Reinigung der Haut und/oder des Haars, bei dem eine kosmetische Zusammensetzung gemäß einem der Ansprüche 1 - 15 auf die Haut und/oder die Haare aufgetragen und anschließend gewünschtenfalls abgespült und/oder abgewischt wird.

## Claims

1. A cosmetic composition, which may be foamed with a post-foaming propellant, containing
a) water,
b) at least one C₁₀-C₂₄ alkane-carboxylic acid,
c) at least one neutralization agent for b),
d) at least one non-ionic surfactant,
e) at least one hydrophobic co-emulsifier,
f) at least one water-soluble C₃-C₉ polyol,
g) at least one thickening polymer, which does not represent any polyethylene glycol, wherein at least one 2-alkylamido-2-methylpropanesulfonic acid polymer and at least one diallyldimethylallylammonium chloride/acrylamide copolymer are not contained at the same time,
h) at least one polyethylene glycol with an average molecular weight of at least 300,000 daltons in an amount from 0.001-0.08% by weight, based on the propellant-free composition,
**characterized in that**
i) silicic acid is contained.

2. The cosmetic composition according to claim 1, **characterized in that** the silicic acid is selected from precipitated silicic acids.

3. The cosmetic composition according to claim 1 or 2, **characterized in that** silicic acid is contained on the whole in an amount from 0.01 to 1% by weight, preferably 0.1 to 0.7% by weight and more preferably 0.2 to 0.5% by weight, each based on the total weight of the propellant-free composition.

4. The cosmetic composition according to any of the preceding claims, **characterized in that** at least one polyethylene glycol is contained with an average molecular weight from at least 300,000 daltons in an amount from 0.005 to 0.03% by weight, based on the propellant-free composition.

5. The cosmetic composition according to any of the preceding claims, **characterized in that** the polyethylene glycol has an average molecular weight from 600,000 daltons to 7 million daltons.

6. The cosmetic composition according to any of the preceding claims, **characterized in that** the non-ionic surfactant is selected from monoethanolamides and diethanolamides of linear C₆-C₂₂ alkane-carboxylic acids and mixtures thereof, from addition products of 1-10 moles of ethylene oxide on amides of linear C₆-C₂₂ alkane carboxylic acids and mixtures thereof as well as addition products of 2-50 moles of ethylene oxide on linear and branched, saturated and unsaturated C₈-C₃₀ alkanols and C₈-C₃₀ alkenols.

7. The cosmetic composition according to any of the preceding claims, **characterized in that** the hydrophobic co-emulsifiers are selected from propylene glycol mono- and di-esters of linear or branched, saturated or unsaturated C₆-C₂₂ carboxylic acids, from glycerol mono- and di-esters of linear or branched, saturated on unsatured C₆-C₂₂ carboxylic acids and from sorbitan mono-, di- and tri-esters of linear or branched, saturated or unsatured C₆-C₂₂ carboxylic acids, which have an HLB value from 1 to 6.

8. The cosmetic composition according to any of the preceding claims, **characterized in that** the thickening polymer g) is selected from chemically modified, if desired, cellulose derivatives, starches, starch degradation products, chemically and/or thermally modified starches, chitosan and its derivatives, polyalkyleneglycol ethers of general formula R-O-(CH₂-CH₂-O)ₙ-R' or R-O-(CH₂-CH(CH₃)-O)ₙ-R' and polyalkyleneglycol esters of general formula R-COO-(CH₂-CH₂-O)ₙ-C(O)-R' or R-COO-(CH₂-CH(CH₃)-O)ₙ-C(O)-R', R and R' representing independently of each other a linear or branched C₆-C₃₀ alkyl or C₆-C₃₀ alkenyl group and n representing a number greater than 75, from synthetic anionic homo- and co-polymers of acrylic acid, methacrylic acid, crotonic acid, maleic anhydride or 2-acrylamido-2-methylpropane-sulfonic acid, synthetic cationic homo- and co-polymers, in which quaternary ammonium groups are bound via a C₁-C₄ hydrocarbon group to a polymer main chain built from acrylic acid, methacrylic acid, or derivatives thereof, inorganic polymer thickening agents, as well as mixtures of these polymers, wherein at least one 2-acrylamido-2-methyl propane sulfonic acid polymer and at last one diallyldimethylallylammonium chloride/acrylamide copolymer are not contained at the same time.

9. The cosmetic composition according to any of the preceding claims, **characterized in that** it is free of polysaccharide gum.

10. The cosmetic composition according to any of the preceding claims, **characterized in that** it contains at least one polyalkyleneglycol ether of general formula R-O-(CH₂-CH₂-O)ₙ-R' or R-O-(CH₂-CH(CH₃)-O)ₙ-R' and/or at least one polyalkyleneglycol ester of general formula R-COO-(CH₂-CH₂-O)ₙ-C(O)-R' or R-COO-(CH₂-CH(CH₃)-O)ₙ-C(O)-R', R and R' representing independently of each other a linear or branched C₆-C₃₀ alkyl or C₆-C₃₀ alkenyl group and n, the average number of alkylene oxide units, represents a number greater than 75, in total amounts from 0.01 to 0.1% by weight, each based on the total weight of the propellant-free composition.

11. The cosmetic composition according to any of the preceding claims, **characterized in that** at least one polyalkyleneglycol-modified silicone is contained.

12. The cosmetic composition according to any of the preceding claims, **characterized in that** at least one oil and/or at least one fat or mixtures thereof are contained.

13. The cosmetic composition according to any of the preceding claims, **characterized in that** at least one skin-cooling active substance is contained.

14. The cosmetic composition according to any of the preceding claims, **characterized in that** it contains a propellant mixture of isopentane and isobutene in the isopentane/isobutene weight ratio from 1:1 to 9:1.

15. The cosmetic composition according to any of the preceding claims, **characterized in that** the weight ratio between the propellant and the propellant-free soap composition is from 20:80 to 1:99.

16. Non-therapeutic use of a cosmetic composition according to any of claims 1 to 15 as a shaving assistant or as a cleaner for the skin and/or the hair.

17. Non-therapeutic use of a cosmetic composition according to any of claims 1 to 15 for moisturizing and/or as a moisturizer and/or for freshening.

18. A non-therapeutic cosmetic method for shaving and/or for cleaning the skin and/or the hair, wherein a cosmetic composition according to any of claims 1 to 15 is applied on the skin and/or the hair and subsequently rinsed and/or wiped dry as desired.

## Revendications

1. Composition cosmétique qui peut être transformée en mousse avec un agent de moussage ultérieur, contenant
a) de l'eau ;
b) au moins un acide alcane(en C₁₀-C₂₄)carboxylique ;
c) au moins un agent de neutralisation pour b) ;
d) au moins un agent tensioactif non ionique ;
e) au moins un co-émulsifiant hydrophobe ;
f) au moins un polyol en C₃-C₉ soluble dans l'eau ;
g) au moins un polymère épaississant, qui ne représente pas un polyéthylèneglycol, au moins un polymère d'acide 2-acrylamido-2-méthylpropanesulfonique et au moins un copolymère de chlorure de diallyldiméthylallylammonium-acrylamide n'étant pas contenus de manière simultanée ;
h) au moins un polyéthylèneglycol possédant un poids moléculaire moyen d'au moins 300.000 Dalton en une quantité de 0,001 à 0,08 % en poids, rapportés à la composition exempte d'agent moussant ;
**caractérisée en ce qu'**elle contient
i) de l'acide silicique.

2. Composition cosmétique selon la revendication 1, **caractérisée en ce que** l'acide silicique est choisi parmi des acides siliciques précipitants.

3. Composition cosmétique selon la revendication 1 ou 2, **caractérisée en ce que** l'acide silicique est contenu au total dans des quantités de 0,01 à 1 % en poids, de préférence de 0,1 à 0,7 % en poids et de manière particulièrement préférée de 0,2 à 0,5 % en poids, chaque fois rapporté au poids total de la composition exempte d'agent moussant.

4. Composition cosmétique selon l'une quelconque des revendications précédentes, **caractérisée en ce qu'**elle contient au moins un polyéthylèneglycol possédant un poids moléculaire moyen d'au moins 300.000 Dalton en une quantité de 0,005 à 0,03 % en poids, rapporté à la composition exempte d'agent moussant.

5. Composition cosmétique selon l'une quelconque des revendications précédentes, **caractérisée en ce que** le polyéthylèneglycol présente un poids moléculaire moyen de 600.000 Dalton à 7 millions de Dalton.

6. Composition cosmétique selon l'une quelconque des revendications précédentes, **caractérisée en ce que** l'agent tensioactif non ionique est choisi parmi des monoéthanolamides et des diéthanolamides d'acides alcane(en C₆-C₂₂)carboxyliques linéaires et leurs mélanges, des produits de fixation par addition de 1 à 10 mol d'oxyde d'éthylène sur les amides d'acides alcane(en C₆-C₂₂)carboxyliques linéaires et leurs mélanges, ainsi que des produits de fixation par addition de 2 à 50 mol d'oxyde d'éthylène sur des alcanols en C₆-C₃₀ et des alcénols en C₆-C₃₀ saturés et insaturés, linéaires et ramifiés.

7. Composition cosmétique selon l'une quelconque des revendications précédentes, **caractérisée en ce que** les co-émulsifiants hydrophobes sont choisis parmi les monoesters et les diesters de propylèneglycol d'acides carboxyliques en C₆-C₂₂ saturés ou insaturés, linéaires ou ramifiés, les monoesters et les diesters de glycérol d'acides carboxyliques en C₆-C₂₂ saturés ou insaturés, linéaires ou ramifiés, et les monoesters, les diesters et les triesters de sorbitanne d'acides carboxyliques en C₆-C₂₂ saturés ou insaturés, linéaires ou ramifiés, qui présentent une valeur HLB de 1 à 6.

8. Composition cosmétique selon l'une quelconque des revendications précédentes, **caractérisée en ce que** le polymère épaississant g) est choisi parmi des dérivés de la cellulose soumis, si on le souhaite, à une modification par voie chimique, des amidons, des produits de dégradation de l'amidon, des amidons modifiés par voie chimique et/ou par voie thermique, du chitosan et ses dérivés, des éthers de polyalkylèneglycols répondant à la formule générale R-O-(CH₂-CH₂-O)ₙ-R' ou R-O-(CH₂-CH(CH₃)-O)ₙ-R' et des esters de polyalkylèneglycols répondant à la formule générale R-COO-(CH₂-CH₂-O)ₙ-C(O)-R' ou R-COO-(CH₂-CH(CH₃)-O)ₙ-C(O)-R', dans lesquelles R et R' représentent, indépendamment l'un de l'autre, un groupe alkyle en C₆-C₃₀ ou un groupe alcényle en C₆-C₃₀ linéaire ou ramifié et n représente un nombre supérieur à 75, des homopolymères et des copolymères anioniques synthétiques de l'acide acrylique, l'acide méthacrylique, de l'acide crotonique, de l'anhydride de l'acide maléique ou de l'acide 2-acrylamido-2-méthylpropanesulfonique, des homopolymères et des copolymères cationiques synthétiques dans lesquels des groupes d'ammonium quaternaires sont liés via un groupe d'hydrocarbure en C₁-C₄ à une chaîne principale polymère constituée d'acide acrylique, d'acide méthacrylique ou de leurs dérivés, des agents épaississants polymères inorganiques ainsi que des mélanges de ces polymères, au moins un polymère d'acide 2-acrylamido-2-méthylpropanesulfonique et au moins un copolymère de chlorure de diallyldiméthylallylammonium-acrylamide n'étant pas contenus de manière simultanée.

9. Composition cosmétique selon l'une quelconque des revendications précédentes, **caractérisée en ce qu'**elle est exempte de gommes de polysaccharides.

10. Composition cosmétique selon l'une quelconque des revendications précédentes, **caractérisée en ce qu'**elle contient au moins un éther de polyalkylèneglycol répondant à la formule générale R-O-(CH₂-CH₂-O)ₙ-R' ou R-O-(CH₂-CH(CH₃)-O)ₙ-R' et/ou au moins un ester de polyalkylèneglycol répondant à la formule générale R-COO-(CH₂-CH₂-O)ₙ-C(O)-R' ou R-COO-(CH₂-CH(CH₃)-O)ₙ-C(O)-R', R et R' représentant, indépendamment l'un de l'autre, un groupe alkyle en C₆-C₃₀ ou un groupe alcényle en C₆-C₃₀ linéaire ou ramifié et n, le nombre moyen des unités d'oxydes d'alkylènes, représentant un nombre supérieur à 75, dans des quantités totales de 0,01 à 0,1 % en poids, chaque fois rapporté au poids total de la composition exempte d'agent moussant.

11. Composition cosmétique selon l'une quelconque des revendications précédentes, **caractérisée en ce qu'**elle contient au moins un silicone modifié avec du polyalkylèneglycol.

12. Composition cosmétique selon l'une quelconque des revendications précédentes, **caractérisée en ce qu'**elle contient au moins une huile et/ou au moins une substance grasse ou leurs mélanges.

13. Composition cosmétique selon l'une quelconque des revendications précédentes, **caractérisée en ce qu'**elle contient au moins une substance active qui refroidit la peau.

14. Composition cosmétique selon l'une quelconque des revendications précédentes, **caractérisée en ce qu'**elle contient un mélange d'agents moussants constitué d'isopentane et d'isobutane dans le rapport pondéral isopentane/isobutane de 1:1 à 9:1.

15. Composition cosmétique selon l'une quelconque des revendications précédentes, **caractérisée en ce que** le rapport pondéral entre l'agent moussant et la composition de savon exempte d'agent moussant s'élève de 20:80 à 1:99.

16. Utilisation non thérapeutique d'une composition cosmétique selon l'une quelconque des revendications 1 à 15, à titre d'adjuvant de rasage ou d'agent de nettoyage pour la peau et/ou pour les cheveux.

17. Utilisation non thérapeutique d'une composition cosmétique selon l'une quelconque des revendications 1 à 15, à des fins d'humidification et/ou à titre d'hydratant et/ou pour le rafraîchissement.

18. Procédé cosmétique, non thérapeutique pour le rasage et/ou pour le nettoyage de la peau et/ou des cheveux, dans lequel on applique une composition cosmétique selon l'une quelconque des revendications 1 à 15 sur la peau et/ou sur les cheveux et ensuite, comme on le souhaite, on l'élimine par rinçage et/ou par essuyage.
